**Europäisches Patentamt**

**European Patent Office**

**Office européen des brevets**

(19)

(11) Veröffentlichungsnummer : **0 429 576 B1**

(12) **EUROPÄISCHE PATENTSCHRIFT**

(45) Veröffentlichungstag der Patentschrift :
28.10.92 Patentblatt 92/44

(51) Int. Cl.⁵ : **A61K 7/08,** A61K 7/50,
A61K 7/06, C11D 1/65

(21) Anmeldenummer : 90907007.0

(22) Anmeldetag : 08.05.90

(86) Internationale Anmeldenummer :
PCT/EP90/00742

(87) Internationale Veröffentlichungsnummer :
WO 90/15589 27.12.90 Gazette 90/29

(54) KLARES HAAR- UND KÖRPERREINIGUNGSMITTEL.

(30) Priorität : 16.06.89 DE 3919669

(43) Veröffentlichungstag der Anmeldung :
05.06.91 Patentblatt 91/23

(45) Bekanntmachung des Hinweises auf die
Patenterteilung :
28.10.92 Patentblatt 92/44

(84) Benannte Vertragsstaaten :
DE ES FR GB IT

(56) Entgegenhaltungen :
EP-A- 0 236 677

(56) Entgegenhaltungen :
Cosmetics & Toiletries, vol. 100, May 1985,
Allured Publishing Corp. T. Schoenberg:
"Formulating mild foaming bathproducts",
pages 53-58
Badekosmetik, Kosmetikjahrbuch 1986, Verlag für chemische Industrie, H. Ziolkowsky KG,
(Augsburg, DE) S. Gati et al.:"Einfluss von
polyquaternaren Additiven auf die Eigenschaften von Shampoos und Flüssigseifen-
Formulierungen bei aussergewöhnlichniedriger Dosierung" pages 50-66

(73) Patentinhaber : **Wella Aktiengesellschaft**
**Berliner Allee 65**
**W-6100 Darmstadt (DE)**

(72) Erfinder : **SCHWARZ, Horst**
**Fliederweg 22 D**
**W-6104 Seeheim (DE)**
Erfinder : **HÖLZEL, Hans**
**Jahnstra e 9**
**W-6101 Fränkisch-Crumbach (DE)**

EP 0 429 576 B1

Jouve, 18, rue Saint-Denis, 75001 PARIS

**Beschreibung**

Die Erfindung betrifft ein klares, mit einem geeigneten anorganischen Salz verdickbares, wasserhaltiges Haar- und Körperreinigungsmittel auf der Basis von Laurylalkoholdiglykolethersulfat oder eines laurylalkoholdiglykolethersulfathaltigen Tensidgemisches, welches eine Kombination aus einem bestimmten polyquarternären Ammoniumsalz und einem Sulfosuccinat enthält.

Haar- und Körperreinigungsmittel enthalten üblicherweise neben den für die Reinigungswirkung ursächlichen Tensiden haar- und hautpflegende Zusätze, die gewährleisten sollen, daß das Haar sowohl in nassem als auch in trockenem Zustand gut kammbar ist, eine verminderte statische Aufladung zeigt und einen weichen und natürlichen Griff besitzt. Zudem soll die Anwendung dieser Mittel ein angenehmes und gepflegtes Hautgefühl erzeugen. Die so behandelte Haut soll sich glatt und weich anfühlen.

Es ist bekannt, in Haar- und Körperreinigungsmitteln kationische Polymere, beispielsweise kationische Cellulosederivate, kationische Chitinderivate oder quarternäre Ammoniumsalze, als Konditionierungsmittel einzusetzen.

Der Einsatz kationischer Polymere in Mitteln für die Reinigung der Haare erleichtert die Entwirrung der mit dem Mittel behandelten Haare sowie deren weitere Behandlung, und verleiht dem Haar Sprungkraft und Glanz. Die kationische Polymere enthaltenden Körperreinigungsmittel erzeugen zudem ein angenehmes und gepflegtes Hautgefühl. Einen starken konditionierenden Effekt auf Haut und Haar üben beispielsweise bestimmte polyquarternäre Ammoniumsalze aus.

Trotz der sehr guten Pflegewirkung der polyquarternären Ammoniumsalze ist ihr Einsatz bei der Herstellung klarer Haar- und Körperreinigungsmittel bisher begrenzt, da ihr Zusatz zu den üblicherweise anionische Tenside enthaltenden Haut- und Körperreinigungsmitteln zu Trübungen führt und damit die Herstellung klarer Produkte behindert. So bilden sich zum Beispiel bei gleichzeitigem Einsatz von Alkylethersulfat und polyquarternären Ammoniumsalzen in wäßriger Lösung unlösliche Trübungen (Vergleichsbeispiel 3, Tab.1). Verwendet man als Tensidgrundlage für Haar- und Körperreinigungsmittel eine Mischung von Alkylethersulfat und einem amphoteren Tensid, zum Beispiel Fettsäurealkylamidobetain, so bleibt ein zugesetztes polyquarternäres Ammoniumsalz klar in Lösung (Vergleichsbeispiel 11, Tab. 1). Versucht man jedoch, diese klare Tensidlösung mit einem geeigneten anorganischen Salz, zum Beispiel Natriumchlorid zu verdicken, kommt es wieder zu Ausfällungen (Vergleichsbeispiel 9, Tab. 1).

Ein Bade- und Duschzusatz, welcher neben Sulfosuccinaten ein bestimmtes polyquarternäres Ammoniumsalz enthält und dessen Viskosität mit Natriumchlorid eingestellt wurde, ist von T. Schoenberg beschrieben worden (T. Schoenberg "Formulating Mild Foaming Bath Products", Cosmetics and Toiletries 100 (5), 53 - 58 (1985)). Bei dem dort beschriebenen Bade- und Duschzusatz handelt es sich jedoch um ein getrübtes Produkt.

Es bestand daher das Bedürfnis nach einem klaren, wasserhaltigen Haar- und Körperreinigungsmittel auf der Basis von Laurylalkoholdiglykolethersulfat, welches gute haar- und hautpflegende Eigenschaften besitzt und mit geeigneten anorganischen Salzen verdickbar ist.

Hierzu wurde nun gefunden, daß ein klares, wasserhaltiges Haar- und Körperreinigungsmittel, mit einem Gehalt an 0,1 bis 50 Gewichtsprozent Laurylalkoholdiglykolethersulfat, welches dadurch gekennzeichnet ist, daß es eine Kombination aus

A) Poly[N-[3-(dimethylammonium)propyl]-N′-[3-(ethylenoxyethylendimethylammonium)propyl]-harnstoffdichlorid]

und

(B) einem Sulfosuccinat

enthält, die Aufgabe in hervorragender Weise löst.

Das mit dem erfindungsgemäßen Haarreinigungsmittel behandelte Haar ist in nassem und trockenem Zustand leicht kämmbar, zeigt eine verminderte statische Aufladung und einen weichen, natürlichen Griff.

Die mit dem erfindungsgemäßen Hautreinigungsmittel behandelte Haut fühlt sich weich und glatt an und vermittelt dem Benutzer ein angenehmes und gepflegtes Hautgefühl.

Das neue Haar- und Körperreinigungsmittel enthält das Laurylalkoholdiglykolethersulfat entweder als alleiniges Tensid oder in Kombination mit weiteren, für diesen Zweck geeigneten Tensiden.

Unter den für die neuen Haar- und Körperreinigungsmittel geeigneten Tensiden seien beispielsweise die folgenden genannt:

a) Die anionischen, oberflächenaktiven Agenzien, wie beispielsweise die Alkali-, Erdalkali-, Ammonium- oder Alkanolaminsalze von Alkansulfonaten, Alkylsulfaten und Alkylethersulfaten, die $C_{12}$ bis $C_{18}$-Alkyl- und insbesondere $C_{12}$ bis $C_{14}$-Alkyl-Sulfatnatriumsalze oder -Triethanolaminsalze, die Natrium- oder Triethanolaminsalze von Lauryl- oder Tetradecylethersulfaten und die Polyethercarbonsäuren;

b) die nichtionischen, oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter

2

Lauryl-, Tetradecyl-, Cetyl-, Oleyl- und Stearylalkohol, allein oder im Gemisch; die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide sowie oxethylierte Sorbitanfettsäureester;

c) die kationischen, oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, Tetradecyltrimethylammoniumchlorid oder -bromid, die Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid. die Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide;

d) die amphoteren oder zwitterionischen, oberflächenaktiven Agenzien wie beispielsweise die Carboxylderivate von Imidazol, die N-Alkylbetaine, die N-Alkylaminobetaine, die N-Alkylsulfobetaine, die N-Alkylaminopropionate, die Alkyldimethylammoniumacetate, die $C_{12}$ bis $C_{18}$-Alkyldimethylcarboxymethylammoniumsalze sowie die Fettsäurealkylamidobetaine, beispielsweise Dimethylcarboxymethylenpropylenamido-stearatbetain.

Das Poly[N-[3-(dimethylammonium)propyl]-N'-[3-(ethylenoxyethylendimethylammonium)propyl]-harnstoffdichlorid (A), welches einen Polymerisationsgrad von 4 bis 8, vorzugsweise 6, aufweist und beispielsweise unter dem Namen Mirapol® A 15 von der Firma Miranol Chemical Company, Inc. vertrieben wird, ist in dem erfindungsgemäßen Mittel in einer Menge von 0,1 bis 5 Gewichtsprozent, vorzugsweise 0,5 bis 1,5 Gewichtsprozent, enthalten.

Das Sulfosuccinat der Komponente (B) ist in dem erfindungsgemäßen Haar- und Hautreinigungsmittel in einer Menge von 1 bis 15 Gewichtsprozent, vorzugsweise 5 bis 15 Gewichtsprozent, enthalten.

Als Sulfosuccinat kommen alle für die Verwendung in Haar- und Hautreinigungsmitteln geeigneten Sulfosuccinate in Betracht. Derartige Sulfosuccinate sind beispielsweise bei G.A. Nowak, "Die kosmetischen Präparate", 2. Auflage, Seiten 227-229, Verlag für chemische Industrie H. Ziolkowsky KG, Augsburg (1984) beschrieben.

Vorzugsweise handelt es sich bei dem Sulfosuccinat um das Dinatriumsalz des vier Oxyethyleneinheiten enthaltenden Kokosfettsäureisopropanolamidhalbesters der Sulfobernsteinsäure oder um das Dinatriumsalz eines ein bis vier Oxyethyleneinheiten enthaltenden Laurylalkoholhalbesters der Sulfobernsteinsäure.

Die dieser Erfindung zugrundeliegende Aufgabe wird besonders gut gelöst, wenn in dem erfindungsgemäßen Mittel als zusätzliches Tensid ein Betaintensid enthalten ist. Als Betaintensid kommen alle für die Verwendung in Haarund Hautreinigungsmitteln geeigneten Betaintenside in Betracht, wobei das Kokosamidopropylbetain besonders bevorzugt ist. Weitere geeignete Betaintenside werden beispielsweise bei H. Janistyn, "Handbuch der Kosmetika und Riechstoffe", I. Band, Seite 989, Dr. A. Hüthig Verlag, Heidelberg (1975) beschrieben.

Das Betaintensid kann in dem erfindungsgemäßen Mittel in einer Menge von 0,1 bis 5 Gewichtsprozent, vorzugsweise in einer Menge von 1 bis 3 Gewichtsprozent, enthalten sein.

Das erfindungsgemäße klare, wasserhaltige Haar- und Körperreinigungsmittel kann zur Einstellung der Viskosität ein physiologisch verträgliches Salz, beispielsweise Natriumsulfat, Kaliumchlorid oder Natriumchlorid, vorzugsweise Natriumchlorid enthalten, ohne daß Trübungen oder Ausfällungen im neuen Mittel auftreten. Die Viskosität des Haar- und Körperreinigungsmittles beträgt 200 bis 5000 mPa·s (Millipascalsekunden), vorzugsweise 1000 bis 3000 mPa·s. Das physiologisch verträgliche Salz wird, je nach der gewünschten Viskosität, in einer Menge von etwa 0,1 bis 6 Gewichtsprozent eingesetzt.

Selbstverständlich kann das erfindungsgemäße Mittel neben den genannten Bestandteilen auch übliche kosmetische Zusätze, beispielsweise Parfümöle in einer Menge von etwa 0,5 bis 5,0 Gewichtsprozent, Verdickungsmittel, wie beispielsweise Kokosfettsäurediethanolamid, in einer Menge von etwa 0,5 bis 10,0 Gewichtsprozent, Verdünnungsmittel, wie zum Beispiel 1,2-Propylenglykol oder ethoxyliertes Sorbitanmonolaurat, in einer Menge von etwa 0,5 bis 5,0 Gewichtsprozent, Puffersubstanzen, wie beispielsweise Natriumcitrat oder Natriumphosphat, in einer Menge von etwa 0,1 bis 1,0 Gewichtsprozent, Lösungsvermittler, wie zum Beispiel ethoxyliertes, gegebenenfalls hydriertes Rizinusöl, in einer Menge von etwa 0,1 bis 1,0 Gewichtsprozent, sowie Farbstoffe, wie zum Beispiel Fluorescein-Natriumsalz, in einer Menge von etwa 0,1 bis 1,0 Gewichtsprozent, enthalten. Außerdem kann es Feuchthaltemittel, Lichtschutzmittel, Antioxidantien, Komplexbildner und Antischuppenwirkstoffe enthalten.

Weitere übliche, für derartige Mittel bekannte Bestandteile, welche in dem neuen Mittel enthalten sein können, sind beispielsweise bei H. Janistyn, "Handbuch der Kosmetika und Riechstoffe", 3. Band, Seiten 228 - 284 und 442 - 462 (1973), sowie bei K. Schrader, "Grundlagen und Rezepturen der Kosmetika", Seiten 375 - 401 und 445 -455 (1979, und ferner bei G. A. Nowak, "Die kosmetischen Präparate", Seiten 452 - 512, (1984) beschrieben.

Die nachfolgenden Beispiele sollen den Gegenstand der Erfindung näher erläutern, ohne ihn jedoch auf die Beispiele zu beschränken.

**Beispiele**

**Beispiel 1: Haarwaschmittel**

| | |
|---|---|
| 40,00 g | Laurylalkohol-diglykolethersulfat, Natriumsalz (28prozentige wäßrige Lösung) |
| 0,70 g | Poly[N-[3-(dimethylammonium)propyl]-N'-[3-(ethylenoxyethylendimethylammonium)propyl]-harnstoffdichlorid] (Polymerisationsgrad = 6, 64prozentige wäßrige Lösung) |
| 5,00 g | Dinatriumsalz des vier Oxyethyleneinheiten enthaltenden Kokosfettsäureisopropanolamid-halbesters der Sulfobernsteinsäure (50prozentige wäßrige Lösung) |
| 4,20 g | Natriumchlorid |
| 50,10 g | Wasser |
| -------- | |
| 100,00 g | |

Die Viskosität des vorstehenden klaren Mittels beträgt 950 mPa·s, gemessen bei 30 Grad Celsius mit einer Haake-Viskowaage unter der Verwendung von Stab II und einem Auflagegewicht von 5 g.

Das mit dem beschriebenen Haarwaschmittel gewaschene Haar ist in nassem und trocknem Zustand leicht kämmbar, zeigt eine verminderte statische Aufladung und besitzt einen weichen, natürlichen Griff.

**Beispiel 2: Duschbad**

| | |
|---|---|
| 35,00 g | Laurylalkohol-diglykolethersulfat, Natriumsalz (28prozentige wäßrige Lösung) |
| 7,00 g | Kokosamidopropylbetain (30prozentige wäßrige Lösung) |
| 1,20 g | Poly[N-[3-(dimethylammonium)propyl]-N'-[3-(ethylenoxyethylendimethylammonium)propyl]-harnstoffdichlorid] (Polymerisationsgrad = 6 64prozentige wäßrige Lösung) |
| 8,00 g | Dinatriumsalz eines ein bis vier Oxyethyleneinheiten enthaltenden Laurylalkoholhalbesters der Sulfobernsteinsäure |
| 1,60 g | Natriumchlorid |
| 47,20 g | Wasser |
| -------- | |
| 100,00 g | |

Die Viskosität des vorstehenden klaren Mittels beträgt 1350 mPa·s, gemessen bei 30 Grad Celsius mit einer Haake-Viskowaage unter Verwendung von Stab II und einem Auflagegewicht von 5 g.

Das erfindungsgemäße Duschbad hinterläßt ein angenehmes und gepflegtes Hautgefühl. Die Haut fühlt sich glatt und weich an.

**Vergleichsbeispiele 3 - 11:**

In der Tabelle 1 ist das Trübungsverhalten der polyquarternäre Ammoniumsalze enthaltenden, nicht erfindungssemäßen Haar- und Körperreinigungsmittel vor und nach dem Andicken mit Natriumchlorid dem der erfindungsgemäßen Zusammensetzungen gegenübergestellt.

## Tabelle 1

| | 3 | 4 | 5 |
|---|---|---|---|
| Laurylalkohol-dialykol-ethersulfat, Natriumsalz (28prozentige wäßrige Lösung) | 35,0 | 35,0 | 35,0 |
| Kokosamidopropylbetain (30prozentige wäßrige Lösung) | - | - | - |
| Poly [N- [3(dimethylammonium)-propyl ]-N'-[3-(ethylenoxy-ethylendimethylammonium)propyl]-harnstoffdichlorid](Polymerisationsgrad = 6, 64prozentige wäßrige Lösung) | 1,0 | 1,0 | 1,0 |
| Dinatriumsalz eines ein bis vier Oxyethyleneinheiten enthaltenden Laurylalkoholhalbesters der Sulfobernsteinsäure | - | - | 5,0 |
| Dinatriumsalz des vier Oxyethyleneinheiten enthaltenden Kokosfettsäure isopropanolamidhalbesters der Sulfobernsteinsäure | - | - | - |
| Natriumchlorid | - | 2,0 | - |
| Wasser | 64,0 | 62,0 | 59,0 |
| | flok-kiger Nie-der-schlag | flok-kiger Nie-der-schlag | klar |

6

## Tabelle 1 (Fortsetzung)

| | 6 | 7 | 8 |
|---|---|---|---|
| Laurylalkohol-dialykol-ethersulfat, Natriumsalz (28prozentige wäßrige Lösung) | 35,0 | 40,0 | 40,0 |
| Kokosamidopropylbetain (30prozentige wäßrige Lösung) | - | - | - |
| Poly [ N- [ 3(dimethylammonium)-propyl ] -N'-[3-(ethylenoxy-ethylendimethylammonium)propyl] -harnstoffdichlorid](Polymerisationsgrad = 6, 64prozentige wäßrige Lösung) | 1,0 | 1,0 | 1,0 |
| Dinatriumsalz eines ein bis vier Oxyethyleneinheiten enthaltenden Laurylalkoholhalbesters der Sulfobernstein-säure | 10,0 | - | - |
| Dinatriumsalz das vier Oxyethyleneinheiten enthaltenden Kokosfettsäure isopropanolamidhalbesters der Sulfobernsteinsäure | - | 5,0 | 5,0 |
| Natriumchlorid | 2,0 | - | 2,0 |
| Wasser | 52,0 | 54,0 | 52,0 |
| | klar | klar | klar |

7

## Tabelle 1 (Fortsetzung)

|  | 9 | 10 | 11 |
|---|---|---|---|
| Laurylalkohol-diglykol-ethersulfat, Natriumsalz (28prozentige wäßrige Lösung) | 35,0 | 35,0 | 35,0 |
| Kokosamidopropylbetain (30prozentige wäßrige Lösung) | 5,0 | 5,0 | 5,0 |
| Poly [N-[3-(dimethylammonium)-propyl] -N'-[3-(ethylenoxy-ethylendimethylammonium)propyl]-harnstoffdichlorid] (Polymerisationsgrad = 6, 64prozentige wäßrige Lösung) | 1,0 | 1,0 | 1,0 |
| Dinatriumsalz eines ein bis vier Oxyethyleneinheiten enthaltenden Laurylalkoholhalb-esters der Sulfobernstein-saure | – | 5,0 | – |
| Dinatriumsalz des vier Oxyethyleneinheiten enthaltenden Kokosfettsäure-isopropanolamidhalbesters der Sulfobernsteinsäure | – | – | – |
| Natriumchlorid | 2,0 | 2,0 | – |
| Wasser | 57,0 | 52,0 | 59,0 |
|  | milchig trüb | klar | klar |

Die Ergebnisse der Vergleichsbeispiele 3 - 11 zeigen, daß im Fall der mit Natriumchlorid verdickten Zubereitungen nur dann ein klares Mittel erhalten wird, wenn das Mittel eine erfindungsgemäße Zusammensetzung aufweist (vergleiche Beispiele 6, 8 und 10).

**Vergleichsbeispiele 12 und 13**

In der Tabelle 2 wird gezeigt, daß, falls in den Vergleichsbeispielen 6 und 8 die erfindungsgemäße polyquarternäre Verbindung Poly[N-[3-(dimethylammonium)-propyl]-N'-[3-(ethylenoxyethylendimethylammonium)propyl]-harnstoffdichlorid], wie es beispielsweise von der Firma Miranol Chemical Company, Inc., New Jersey unter dem Handelsnamen Mirapol ® A15 vertrieben wird, durch ein Copolymer aus Dimethyldiallylammoniumchlorid und Acrylamid, wie es beispielsweise von der Firma Merck & Co., Inc., New Jersey unter dem Handelsnamen Merquat ® 550 vertrieben wird, mengengleich (bezogen auf die Aktivsubstanz) ersetzt wird, keine klaren Mittel erhalten werden:

## Tabelle 2

|  | 12 | 13 |
|---|---|---|
| Laurylalkohol-diglykol-ethersulfat, Natriumsalz (28prozentige wäßrige Lösung) | 35,0 | 40,0 |
| Kokosamidopropylbetain (30prozentige wäßrige Lösung) | – | – |
| Copolymer aus Dimethyl-diallylammoniumchlorid und Acrylamid (8prozentige wäßrige Lösung) | 8,0 | 8,0 |
| Dinatriumsalz eines ein bis vier Oxyethyleneinheiten enthaltenden Laurylalkoholhalb-esters der Sulfobernstein-säure | 10,0 | – |
| Dinatriumsalz des vier Oxyethyleneinheiten enthaltenden Kokosfettsäure-isopropanolamidhalbesters der Sulfobernsteinsäure | – | 5,0 |
| Natriumchlorid | 2,0 | 2,0 |
| Wasser | 45,0 | 45,0 |
|  | trüb | trüb |

Sämtliche in der Anmeldung angegebenen Prozentzahlen stellen Gewichtsprozente dar.

**Patentansprüche**

1. Klares, wasserhaltiges Haar- und Körperreinigungsmittel mit einem Gehalt an 0,1 bis 50 Gewichtsprozent Laurylalkoholdiglykolethersulfat, dadurch gekennzeichnet, daß es eine Kombination aus
   (A) Poly[N-[3-(dimethylammonium)propyl]-N'-[3-(ethylenoxyethylendimethylammonium)propyl]-harnstoffdichlorid]
             und
   (B) einem Sulfosuccinat
       enthält.

2. Mittel nach Anspruch 1, dadurch gekennzeichnet, daß die Komponente (A) in einer Menge von 0,1 bis 5 Gewichtsprozent, enthalten ist.

3. Mittel nach Anspruch 1 oder 2, dadurch gekennzeichnet, daß die Komponente (B) in einer Menge von 1 bis 15 Gewichtsprozent, enthalten ist.

4. Mittel nach einem der Ansprüche 1 bis 3, dadurch gekennzeichnet, daß es sich bei der Komponente (B) um das Dinatriumsalz des vier Oxyethyleneinheiten enthaltenden Kokosfettsäureisopropanolamidhalbesters der Sulfobernsteinsäure oder um das Dinatriumsalz eines ein bis vier Oxyethyleneinheiten enthaltenden Laurylalkoholhalbesters der Sulfobernsteinsäure handelt.

5. Mittel nach einem der Ansprüche 1 bis 4, dadurch gekennzeichnet, daß es zur Einstellung der Viskosität des Mittels ein physiologisch verträgliches Salz enthält.

6. Mittel nach Anspruch 5, dadurch gekennzeichnet, daß das physiologisch verträgliche Salz Natriumchlorid ist.

7. Mittel nach einem der Ansprüche 1 bis 6, dadurch gekennzeichnet, daß es eine Viskosität von 200 bis 5000 mPa·s (Millipascalsekunden) aufweist.

8. Mittel nach einem der Ansprüche 1 bis 7, dadurch gekennzeichnet, daß es ein Betaintensid enthält.

9. Mittel nach Anspruch 8, dadurch gekennzeichnet, daß das Betaintensid Kokosamidopropylbetain ist.

10. Mittel nach einem der Ansprüche 8 oder 9 dadurch gekennzeichnet, daß das Betaintensid in einer Menge von 0,1 bis 5 Gewichtsprozent enthalten ist.

**Claims**

1. Clear, aqueous hair and body washing agent containing 0.1 to 50 weight percent of lauryl alcohol diglycolethersulphate, characterised in that it contains a combination of:
   (A) poly[N-[3-(dimethylammonium)propyl]-N'-[3-(ethyleneoxy-ethylenedimethyl-ammonium)propyl]-urea dichloride];
   and
   (B) a sulphosuccinate.

2. Agent according to Claim 1, characterised in that component (A) is present in an amount of 0.1 to 5 weight percent.

3. Agent according to Claim 1 or 2, characterised in that component (B) is present in an amount of 1 to 15 weight percent.

4. Agent according to one of Claims 1 to 3, characterised in that component (B) is the disodium salt of the coconut fatty acid isopropanolamide half-ester of sulphosuccinic acid, containing four ethylene oxide units, or the disodium salt of a lauryl alcohol half-ester of sulphosuccinic acid, containing one to four ethylene oxide units.

5. Agent according to any one of Claims 1 to 4, characterised in that it contains a physiologically compatible salt in order to adjust the viscosity of the agent.

6. Agent according to Claim 5, characterised in that the physiologically compatible salt is sodium chloride.

7. Agent according to any one of Claims 1 to 6, characterised in that it has a viscosity of between 200 and 5000 mPa.s (millipascalseconds).

8. Agent according to any one of Claims 1 to 7, characterised in that it contains a betaine surfactant.

9. Agent according to Claim 8, characterised in that the betaine surfactant is coconut amidopropylbetaine.

10. Agent according to either of Claims 8 or 9, characterised in that the betaine surfactant is present in an amount of between 0.1 and 5 weight percent.

## Revendications

1. - Agent de nettoyage du corps et des cheveux, clair et contenant de l'eau, contenant de 0,1 à 50% en poids d'un éther-sulfate d'alcool laurylique et de diglycol, caractérisé en ce qu'il contient une combinaison de
    (A) dichlorure de poly[N-[3-(diméthylammonium)propyl]-N'-[3-(éthylène-oxyde-diméthylammonium) propyl]-urée],
        et
        (B) un sulfosuccinate.

2. - Agent selon la revendication 1, caractérisé en ce que le composant (A) est contenu à raison de 0,1 à 5% en poids.

3. - Agent selon la revendication 1 ou 2, caractérisé en ce que le composé (B) est contenu à raison de 1 à 15% en poids.

4. - Agent selon l'une des revendications 1 à 3, caractérisé en ce qu'il s'agit pour le composant (B) du sel disodique de l'hémiester de l'acide du coprah et de l'isopropylamide de l'acide sulfosuccinique contenant quatre motifs oxyde d'éthylène ou du sel disodique d'un hémiester d'alcool laurylique de l'acide sulfosuccinique contenant un à quatre motifs oxyde d'éthylène.

5. - Agent selon l'une des revendications 1 à 4, caractérisé en ce que pour l'ajustement de la viscosité, l'agent contient un sel physiologiquement compatible.

6. - Agent selon la revendication 5, caractérisé en ce que le sel physiologiquement compatible est le chlorure de sodium.

7. - Agent selon l'une des revendications 1 à 6, caractérisé en ce qu'il présente une viscosité de 200 à 5000 mPa·sec (millipascal.seconde).

8. - Agent selon l'une des revendications 1 à 7, caractérisé en ce qu'il contient un agent tensio-actif à base de bétaïne.

9. - Agent selon la revendication 8, caractérisé en ce que l'agent tensio-actif à base de bétaïne est l'amidopropyl-bétaïne de l'acide du coprah.

10.- Agent selon la revendication 8 ou 9, caractérisé en ce que l'agent tensio-actif à base de bétaïne est présent à raison de 0,1 à 5% en poids.